# EUROPÄISCHE PATENTANMELDUNG

(11) **EP 3 841 938 A1**
(43) Veröffentlichungstag der Anmeldung: **30.06.2021**
(21) Anmeldenummer: 20207572.7
(22) Anmeldetag: 13.11.2020
(51) Int. Cl.: A47K 5/12, A47K 10/32, G01F 15/06, G06Q 10/06, G16H 40/20

(54) **VORRICHTUNG UND VERFAHREN ZUR ABGABE EINES TUCHS ODER EINER REINIGUNGSFLÜSSIGKEIT**

(30) Priorität: 23.12.2019 DE 102019135750
(71) Anmelder: WELLGO Gerätetechnik GmbH, 66625 Nohfelden (DE)
(72) Erfinder: Mechenbier, Jürgen, 66564 Ottweiler (DE); Mechenbier, Thorsten, 66564 Ottweiler (DE); Lenhard, Michael, 66299 Friedrichsthal (DE); Haser, Nico, 66280 Sulzbach (DE)
(74) Vertreter: Patentanwälte Bernhardt / Wolff Partnerschaft mbB

(57) **Zusammenfassung**

Die Erfindung betrifft eine Vorrichtung zur Abgabe eines Tuchs und/oder einer Flüssigkeit, die dazu vorgesehen ist, eine Information zu einer in der Vorrichtung (1) angeordneten oder/und mittels der Vorrichtung (1) abgegebenen Menge des Tuchs und/oder der Flüssigkeit zu speichern. Erfindungsgemäß ist die Vorrichtung (1) dazu vorgesehen, die Information derart zu speichern, dass sie nach der Speicherung nicht veränderbar ist. In einer Ausgestaltung der Erfindung ist die Vorrichtung (1) zur Speicherung der Information in einem, vorzugsweise elektronischen, lediglich einmalig beschreibbaren Kontrolldatenspeicher (2) vorgesehen, wobei der Kontrolldatenspeicher (2) vorzugsweise Teil eines Funkchips (3), vorzugsweise eines RFID-Transponders, ist. Zweckmäßigerweise ist die Vorrichtung (1) dazu vorgesehen, die Informationen zu der abgegebenen Menge in einem Vergleichsdatenspeicher (4) zu speichern, der vorzugsweise lösch- und wiederbeschreibbar ist. In einer Ausführungsform der Erfindung ist die Vorrichtung (1) dazu vorgesehen ist, die in dem Kontrolldatenspeicher (2) und in dem Vergleichsdatenspeicher (4) gespeicherte Information zu der abgegebenen Menge des Tuchs und/oder der Flüssigkeit automatisch miteinander zu vergleichen. Vorzugsweise die Vorrichtung (1) dazu vorgesehen ist, in dem Fall, dass sich die Information des Kontrolldatenspeichers (2) und des Vergleichsdatenspeicher (4) einander nicht entsprechen, eine Abgabe des Tuchs und/oder der Flüssigkeit mittels der Vorrichtung (1) zu verhindern oder die in dem Kontrolldatenspeicher (2) gespeicherte Information zu der Menge in dem Vergleichsdatenspeicher (4) zu speichern.

Die Erfindung betrifft ferner einen Tuch- oder Flüssigkeitsträger (5) zur Anordnung in der Vorrichtung (1) und ein Verfahren zur Abgabe des Tuchs oder der Flüssigkeit.

## Beschreibung

Die Erfindung betrifft eine Vorrichtung zur Abgabe eines Tuchs, insbesondere eines Handtuchs, oder einer Reinigungsflüssigkeit, insbesondere von Seife oder Desinfektionsmittel, die dazu vorgesehen ist, eine Information zu einer in der Vorrichtung angeordneten oder/und mittels der Vorrichtung abgegebenen Menge des Tuchs oder der Reinigungsflüssigkeit zu speichern.

Die Erfindung betrifft ferner einen Träger für das Tuch oder die Reinigungsflüssigkeit zur Anordnung in der Vorrichtung und ein Verfahren zur Abgabe des Tuchs oder der Reinigungsflüssigkeit.

EP 1 956 954 B1 beschreibt eine solche Spendervorrichtung zur Abgabe von Blattmaterial, das auf einer in der Spendervorrichtung austauschbaren Rolle angeordnet ist. Die Spendervorrichtung speichert auf einem an der Rolle angeordneten Chip die Länge des von der Rolle abgegebenen Blattmaterials, damit festgestellt werden kann, wenn auf der Rolle kein Blattmaterial mehr vorhanden ist.

Der Erfindung liegt die Aufgabe zugrunde, zu verhindern, dass die gespeicherte Information manipuliert wird.

Erfindungsgemäß wird diese Aufgabe dadurch gelöst, dass die Vorrichtung dazu vorgesehen ist, die Information derart zu speichern, dass sie nach der Speicherung nicht veränderbar ist.

Eine Änderung der Information, die vorzugsweise auf einem Kontrolldatenspeicher eines in der Vorrichtung vorzusehenden Tuch- oder Reinigungsflüssigkeitsträgers gespeichert wird, von dem aus das Tuch oder die Flüssigkeit abgegeben wird, ist dank der Erfindung nicht möglich.

Vorteilhaft kann dadurch, anders als bei der bekannten Vorrichtung, eine Verwendung des Tuch- oder Flüssigkeitsträgers, wenn er unberechtigt mit dem Tuch oder der Reinigungsflüssigkeit versehen worden ist, ausgeschlossen werden. Bei der bekannten Vorrichtung wird die Information in einen veränderbaren, insbesondere lösch- und wiederbeschreibbaren, Datenspeicher des Chips hinterlegt, der neben der Länge des abgegebenen Blattmaterials auch Informationen über die Identität des Chips sowie die Qualität des Blattmaterials enthält. Bei der bekannten Vorrichtung ist es deshalb möglich, die gespeicherte Information zur Länge des Blattmaterials zu verändern, um den Chip, z.B. mit einer anderen oder einer erneut befüllten Rolle, wiederzuverwenden.

Der genannte Tuch- oder Flüssigkeitsträger, an dem der Kontrolldatenspeicher vorzugsweise angeordnet ist, kann eine Rolle, z.B. aus Pappe oder Kunststoff, sein, auf welcher das Tuch aufgerollt ist, oder ein Flüssigkeitsbehälter, in dem die Reinigungsflüssigkeit angeordnet ist, insbesondere eine Flasche, ein Spritzenkolben oder ein Fass.

Das abzugebende Tuch kann beispielsweise ein Handtuch, insbesondere Papier- oder Stoffhandtuch, sein. Die Reinigungsflüssigkeit ist vorzugsweise Seife oder Desinfektionsmittel.

Die Vorrichtung ist zweckmäßigerweise dazu eingerichtet, den Tuch- oder Flüssigkeitsträger mechanisch zu halten. Sie ist dazu vorzugsweise mit entsprechenden Haltemitteln versehen. Bevorzugt ist die Vorrichtung zur lösbaren Befestigung des Tuch- oder Flüssigkeitsträgers vorgesehen.

In einer Ausführungsform der Erfindung umfasst die Vorrichtung einen Dosierspender, vorzugsweise einen Seifen- und/oder Desinfektionsmittelspender, oder einen Handtuchspender, vorzugsweise einen Papierhandtuch- oder einen Stoffhandtuchspender.

Der Tuch- oder Flüssigkeitsträger ist zweckmäßigerweise zur, vorzugsweise lösbar befestigten, Anordnung in der Vorrichtung eingerichtet. Er kann dazu mit Haltemitteln versehen sein, die die Anordnung, insbesondere Befestigung, in der Vorrichtung ermöglichen.

Die Vorrichtung ist zweckmäßigerweise dazu eingerichtet, die Information über eine Verbindung zur Datenübertragung zu speichern, wenn der Tuch- oder Flüssigkeitsträger in der Vorrichtung angeordnet ist. Die Datenübertragungsverbindung kann über Funk, z.B. per Nahfeldkommunikation ("Near Field Communication", abgekürzt NFC), oder/und über eine Datenleitung erfolgen. Erfolgt die Information über eine Datenleitung, werden Datenleitungsabschnitte der Vorrichtung und des Tuch- oder Flüssigkeitsträgers vorzugsweise miteinander verbunden, wenn der Tuch- oder Flüssigkeitsträger in der Vorrichtung angeordnet wird, beispielsweise über Stecker oder dergleichen.

Um den Speichervorgang durchzuführen, ist die Vorrichtung zweckmäßigerweise mit einem Rechner versehen, vorzugsweise einem Prozessor.

Zweckmäßigerweise weist die Vorrichtung eine Einrichtung zur Messung der abgegebenen Menge des Tuchs oder der Reinigungsflüssigkeit auf. Ist das Tuch auf einer Rolle angeordnet, kann die abgegebene Menge z.B. anhand der Drehung der Rolle ermittelt werden. Ist das abzugebende Tuch als eine Vielzahl von Einzelblättern vorgesehen, kann die Menge durch Zählen der abgegebenen Einzelblätter gemessen werden. Wird die Reinigungsflüssigkeit mittels einer Pumpe ausgegeben, lässt sich die Menge anhand des abgegebenen Pumpvolumens, insbesondere in Abhängigkeit von den durchgeführten Pumpvorgängen, bestimmen. Alternativ könnte auch die Masse des Tuchs oder der Flüssigkeit auf dem Tuch- oder Flüssigkeitsträger gemessen werden und in Abhängigkeit von der Massenänderung die abgegebene Menge ermittelt werden.

In einer Ausgestaltung der Erfindung ist der, vorzugsweise elektronische, Kontrolldatenspeicher lediglich einmalig beschreibbar. Der Kontrolldatenspeicher könnte beispielsweise ein One-Time-Programmable-Baustein sein.

In einer Ausführungsform der Erfindung wird der Kontrolldatenspeicher mit Abgabe des Tuchs oder der Reinigungsflüssigkeit sukzessive beschrieben. Vorzugsweise werden jeweilig nach Abgabe einer vorgegebenen Menge des Tuchs oder der Reinigungsflüssigkeit, beispielsweise nach sukzessiver Abgabe jeweilig einer vorgegebenen Länge an Tuch oder Masse des Tuchs oder eines vorgegebenen Volumens oder einer vorgegebenen Masse der Reinigungsflüssigkeit Daten in den Kontrolldatenspeicher geschrieben. Die Menge wird vorzugsweise mittels der oben genannten Messeinrichtung bestimmt.

Eine Möglichkeit ist, die jeweilig abgegebene Menge oder die jeweilig verbleibende Menge sukzessive in den Kontrolldatenspeicher zu schreiben, besonders bevorzugt jedes Mal, wenn jeweilig die vorgegebene Menge abgegeben worden ist.

In einer bevorzugten Ausführungsform der Erfindung wird jeweilig nach der Abgabe der vorgegebenen Menge eine Dateneinheit, bspw. ein Bit eines Binärsystems, des Kontrolldatenspeichers, bspw. mit der Binärzahl "1", beschrieben. Wird die Anzahl der beschriebenen Dateneinheiten mit der Menge an abgegebenem Tuch oder Reinigungsflüssigkeit verknüpft, lässt sich anhand dieser Information aus dem Kontrolldatenspeicher die von Tuch- oder Flüssigkeitsträger abgegebene oder auf dem Tuch- oder Flüssigkeitsträger verbleibende Menge bestimmen. Vorzugsweise wird die Datenspeicherung auf dem Kontrolldatenspeicher vorgesehen derart, dass der Kontrolldatenspeicher vollständig beschrieben ist, wenn das Tuch oder die Reinigungsflüssigkeit vollständig oder zumindest nahezu vollständig von dem Tuch- oder Flüssigkeitsträger abgegeben ist. Wenn der Kontrolldatenspeicher voll beschrieben ist, blockiert die Vorrichtung jede weitere Abgabe von dem Tuch- oder Flüssigkeitsträger.

Es versteht sich, dass bei vollständiger Entleerung des Tuch- oder Flüssigkeitsträgers der Kontrolldatenspeicher nicht unbedingt vollständig beschrieben werden muss, sondern alternativ auch ein anderer Anteil an beschriebenem Speicherplatz vorgesehen werden könnte, z.B. 80 oder 90 %.

In einer bevorzugten Ausgestaltung der Erfindung ist die Vorrichtung dazu vorgesehen, die Informationen zu der abgegebenen Menge ferner in einem Vergleichsdatenspeicher zu speichern, der vorzugsweise lösch- und wiederbeschreibbar ist.

Die Speicherung der Information kann in dem Vergleichsdatenspeicher in derselben oder auf verschiedene Weise vorgenommen werden wie in dem Kontrolldatenspeicher. Die Speicherung in verschiedener Weise kann vorteilhaft sein, wenn der Vergleichsdatenspeicher eine größere Kapazität zur Speicherung der Information aufweist als der Kontrolldatenspeicher und deshalb die abgegebene bzw. verbleibende Menge in kleineren Schritten gespeichert werden kann als auf dem Kontrolldatenspeicher.

In einer weiteren Ausgestaltung der Erfindung ist die Vorrichtung dazu vorgesehen, die in dem Kontrolldatenspeicher und in dem Vergleichsdatenspeicher gespeicherte Information zu der abgegebenen Menge automatisch, vorzugsweise mittels des genannten oder eines anderen Prozessors, miteinander zu vergleichen. Wird in dem Kontrolldatenspeicher und dem Vergleichsdatenspeicher dieselbe Information hinterlegt, lassen sich die Informationen direkt miteinander vergleichen.

Wird, wie nach einer Ausführungsform der Erfindung vorgesehen, die abgegebene Menge oder die jeweilig verbleibende Menge z.B. als Mengenangabe, sukzessive in den Vergleichsdatenspeicher geschrieben und der Kontrolldatenspeicher wie oben erläutert jeweilig nach der Abgabe der vorgegebenen Menge mit einer Dateneinheit beschrieben, kann die Vorrichtung dazu vorgesehen sein, unter Durchführung eines Algorithmus zu prüfen, ob die Information in dem Kontrolldatenspeicher und diejenige in dem Vergleichsdatenspeicher einander entsprechen, d.h. zueinander passende Informationen über die Abgabe enthalten.

Wird bei dem Vergleich festgestellt, dass die Information in dem Kontrolldatenspeicher nicht derjenigen in dem Vergleichsdatenspeicher entspricht, folgt daraus, dass die Information im Vergleichsdatenspeicher manipuliert worden ist.

Die Vorrichtung ist zweckmäßigerweise dazu vorgesehen, in dem Fall, dass sich die Mengenabgabeinformationen des Kontrolldatenspeichers und des Vergleichsdatenspeichers nicht entsprechen, eine Abgabe des Tuchs oder der Reinigungsflüssigkeit mittels der Vorrichtung zu verhindern oder eine der in dem Kontrolldatenspeicher gespeicherten Information entsprechende Information zu der Menge aus des Tuchs oder der Reinigungsflüssigkeit in den Vergleichsdatenspeicher zu schreiben.

Im erstgenannten Fall kann unter Verwendung des Kontrolldatenspeichers kein Tuch oder keine Reinigungsflüssigkeit mehr abgegeben werden. Im zweitgenannten Fall kann unter Verwendung des Kontrolldatenspeichers lediglich die Menge des Tuchs oder der Reinigungsflüssigkeit abgegeben werden, zu der auf dem Kontrolldatenspeicher noch kein Speicherplatz verbraucht ist.

In einer Ausführungsform der Erfindung ist der Kontrolldatenspeicher und ggf. der Vergleichsdatenspeicher Teil eines RFID-Transponders. Die Vorrichtung umfasst zum Lesen und/oder Schreiben des RFID-Transponders zweckmäßigerweise ein RFID-Schreib- und/oder -lesegerät. Sie ist vorzugsweise dazu eingerichtet, die Information in den Kontrolldatenspeicher und ggf. den Vergleichsdatenspeicher zu schreiben.

In einer Ausgestaltung der Erfindung ist die Vorrichtung dazu vorgesehen, aus dem Vergleichsdatenspeicher und/oder dem Kontrolldatenspeicher eine Kennung auszulesen, anhand derer überprüfbar ist, ob der Tuch- oder Flüssigkeitsträger zur Verwendung in der Vorrichtung autorisiert ist.

Optional kann die Kennung auch Informationen über das Tuch oder die Reinigungsflüssigkeit enthalten, beispielsweise über die Art, die Qualität und/oder die Menge an auf bzw. in dem Tuch- oder Flüssigkeitsträger angeordnetem Tuch bzw. Reinigungsflüssigkeit.

Zweckmäßigerweise wird die Kennung und ggf. auch die gespeicherte Information mittels einer Identifizierung des RFID-Transponders ("ID") verschlüsselt.

Die Erfindung wird nachfolgend anhand von Ausführungsbeispielen und den beigefügten Zeichnungen, die sich auf die Ausführungsbeispiele beziehen, näher erläutert. Es zeigen:
- Fig. 1: eine erfindungsgemäße Vorrichtung in verschiedenen Ansichten,
- Fig. 2: schematisch den Aufbau von Bauteilen der erfindungsgemäßen Vorrichtung nach Fig. 1,
- Fig. 3: eine weitere erfindungsgemäße Vorrichtung in verschiedenen Ansichten, und
- Fig. 4: schematisch den Aufbau von Bauteilen der erfindungsgemäßen Vorrichtung nach Fig. 3.

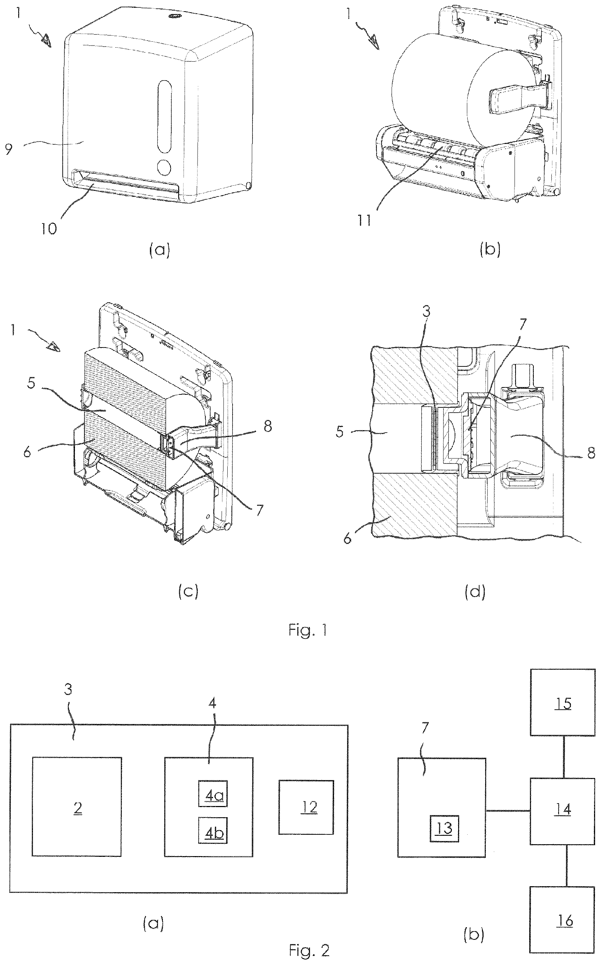

Bei einem in Fig. 1 gezeigten Ausführungsbeispiel ist die erfindungsgemäße Vorrichtung 1 als Papierhandtuchspender ausgebildet. Fig. 1a zeigt ein Gehäuse 9 der Vorrichtung 1, in der eine Ausgabeöffnung 10 vorgesehen ist, durch die hinaus Papierhandtuch ausgegeben wird. Wie Figuren 1b und c zeigen, ist in dem Gehäuse 9 an einem Rollenhalter 8 als Handtuchträger 5 eine Tragrolle angeordnet, auf der das Papierhandtuch 6 aufgerollt ist. Zum Ausgeben des Papierhandtuchs 6 ist ein Antrieb 11 vorgesehen, mit dem das Papierhandtuch 6 gemeinsam mit dem Handtuchträger 5 in Drehung versetzt werden kann.

Fig. 1d zeigt in einem Schnitt durch den Rollenhalter 8 und den Handtuchträger 5, dass in dem Rollenhalter 8 ein RFID-Schreib-/Lesegerät 7 angeordnet ist. In der Tragrolle 5 sitzt ein RFID-Transponder 3, der sich mit dem RFID-Schreib-/Lesegerät 7 auslesen und beschreiben lässt.

In dem Rollenhalter 8 ist ferner eine hier lediglich schematisch in Fig. 2b gezeigte Messeinrichtung 15 gebildet, mittels derer sich die Anzahl der Drehungen des Handtuchträgers 5 in der Vorrichtung 1 messen lässt. Anhand der Anzahl der Drehungen kann die Länge an ausgegebenem Papierhandtuch 6 mittels eines Rechners 14 der Vorrichtung 1 bestimmt und in einem Datenspeicher 16 der Vorrichtung 1 gespeichert werden.

In Fig. 2a ist schematisch gezeigt, dass der RFID-Transponder 3 einen Kontrolldatenspeicher 2 aufweist, der lediglich ein einziges Mal beschrieben und nicht gelöscht oder wiederbeschrieben werden kann. Bei dem Kontrolldatenspeicher 2 kann es sich um einen Festwertspeicher, insbesondere einen elektronischen One-Time-Programmable-Baustein, handeln.

Der RFID-Transponder 3 weist ferner einen weiteren, lösch- und wiederbeschreibbaren Datenspeicher 4 auf. In einem Datenspeicherbereich 4a des Datenspeichers 4 ist eine Kennung des RFID-Transponders 3 hinterlegt, anhand derer mittels des RFID-Schreib-/Lesegeräts 7 durch den Rechner 14 der Vorrichtung 1 ausgelesen werden kann, ob der Handtuchträger 5 zur Verwendung in der Vorrichtung 1 autorisiert ist.

Die Datenübertragung zwischen dem RFID-Schreib-/Lesegerät 7 und RFID-Transponder 3 erfolgt per Funk über eine Antenne 12 des RFID-Transponders 3 und einer Antenne 13 des RFID-Schreib-/Lesegeräts 7.

Bei dem nachfolgend erläuterten Beispiel ist auf dem Handtuchträger 5 Papierhandtuch 6 in einer Länge von 320 m und als Kontrolldatenspeicher 2 ein 32 Bit-Speicher vorgesehen.

Bei Ausgabe des Papierhandtuchs 6 aus der Vorrichtung 1 wird mittels der Messeinrichtung 15, z.B. anhand von Drehungen des Handtuchträgers 5 in der Vorrichtung, die Länge des ausgegebenen Papierhandtuchs 6 bestimmt. Immer wenn 10 m des Papierhandtuchs 6 ausgegeben worden sind, wird mittels des RFID-Schreib-/ Lesegeräts 7 jeweilig ein Bit des Kontrolldatenspeichers 2 mit der Zahl "1" beschrieben. Aus dem Kontrolldatenspeicher 2 lässt sich mit einer Genauigkeit von 10 m, z.B. mittels des Rechners 14, die ausgegebene Papierhandtuchlänge ermitteln. Insbesondere ist das Papierhandtuch 6 vollständig von dem Handtuchträger 5 abgegeben, wenn die 32 Bit des Kontrolldatenspeichers 2 vollständig beschrieben sind.

Der Rechner 14 der Vorrichtung 1 ist so programmiert, dass eine Ausgabe des Papierhandtuchs 6 lediglich dann zugelassen wird, wenn der Kontrolldatenspeicher 2 nicht vollständig beschrieben ist.

Da die Vorrichtung 1 den Kontrolldatenspeicher 2 nach sukzessiver Ausgabe von je 10 m des Papierhandtuchs 6 immer wieder beschreibt, können unter Verwendung des mit dem RFID-Transponders 3 versehenen Handtuchträgers 5 folglich lediglich die genannten 320 m des Papierhandtuchs 6 ausgegeben werden. Selbst wenn der Handtuchträger 5 nach Abgabe des Papierhandtuchs 6 wieder mit neuem Papierhandtuch bestückt würde, könnten nicht mehr als die 320 m von dem Handtuchträger 5 abgegeben werden. Vorteilhaft wird dadurch verhindert, dass unautorisiert auf den Handtuchträger 5 aufgegebenes Papierhandtuch mittels der Vorrichtung 1 abgegeben werden kann.

In einem weiteren Ausführungsbeispiel wird zusätzlich zu der Speicherung im Kontrolldatenspeicher 2 in einem Vergleichsdatenspeicher 4b, der Datenspeicherbereich des Weiteren, lösch- und wiederbeschreibbaren Datenspeichers 4 ist, die Information über die Länge des abgegebenen Papierhandtuchs 6 gespeichert. Um die Länge des abgegebenen Papierhandtuchs 6 genauer bestimmen zu können, wird in den Vergleichsdatenspeicher 4b jeweilig nach Ausgabe von 2 m des Papierhandtuchs 6 eine entsprechend Information gespeichert.

Der Rechner 14 der Vorrichtung 1 ist dazu vorgesehen, mittels eines Algorithmus die Information aus dem Kontrolldatenspeicher 2 mit derjenigen des Vergleichsdatenspeichers 4b zu vergleichen. Sollten die daraus bestimmbaren Längeninformationen einander entsprechen, d.h. die Längeninformation aus dem Vergleichsdatenspeicher 4b, die in 2 m Schritten angelegt worden ist, liegt innerhalb der in 10 m Schritten vorgesehenen Längeninformation aus dem Kontrolldatenspeicher 2, wird eine Papierhandtuchausgabe mittels der Vorrichtung 1 freigegeben. Andernfalls, d.h. wenn die Information im Vergleichsdatenspeicher 4b manipuliert worden ist, wird eine Ausgabe blockiert.

Alternativ kann vorgesehen sein, dass in dem Fall, dass die Information aus dem Kontrolldatenspeicher 2 derjenigen aus dem Vergleichsdatenspeicher 4b entspricht, eine der Information aus dem Kontrolldatenspeicher 2 entsprechende Längeninformation in den Vergleichsdatenspeicher 4b geschrieben wird. Von dem Handtuchträger 5 kann dann noch so viel Papierhandtuch 6 abgegeben werden, wie anhand der Information aus dem Kontrolldatenspeicher 2 vorgesehen ist.

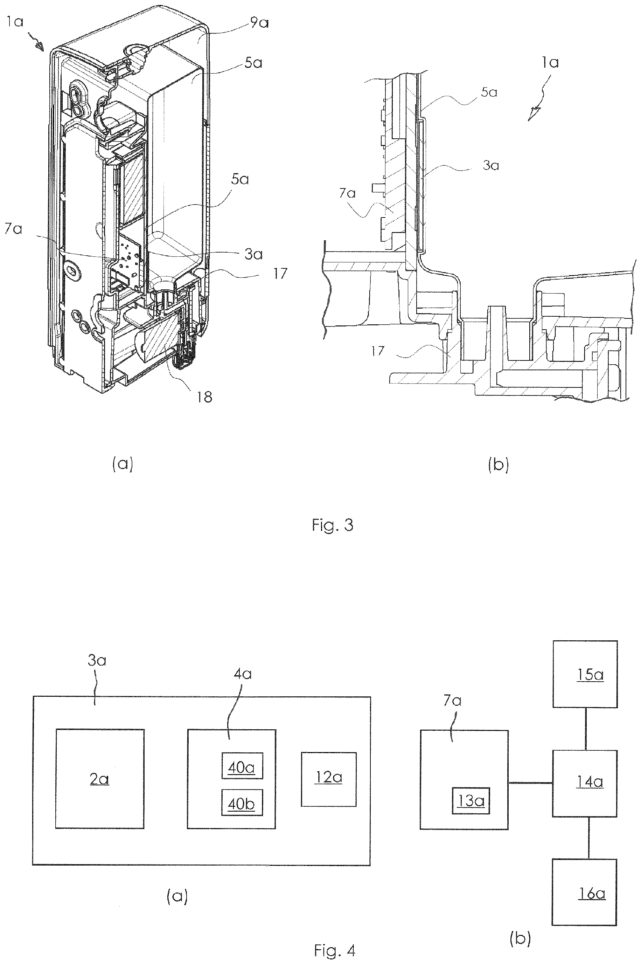

Fig. 3 zeigt als weitere erfindungsgemäße Vorrichtung 1a einen Flüssigkeitsspender, der zur Abgabe von Seife oder von Desinfektionsmittel vorgesehen ist. Die Vorrichtung weist ein Gehäuse 9a auf, das einen Bereich zur Aufnahme eines Flüssigkeitsbehälters 5a aufweist. Der Flüssigkeitsbehälter 5a ist mit einem Auslass 19 versehen, durch den hindurch die Flüssigkeit abgegeben werden kann. Zum kontrollierten Ausgeben der Flüssigkeit ist eine Pumpe, z.B. eine Zahnrad-, Schlauch-, oder Kolbenpumpe, vorgesehen, die sich mittels eines Pumpenmotors 18 betätigen lässt.

Im Inneren des Gehäuses 9a ist ein RFID-Schreib-/Lesegerät 7a angeordnet, neben dem ein RFID-Transponder 3a des Flüssigkeitsbehälters 5a angeordnet ist, wenn der Flüssigkeitsbehälter 5a in dem Gehäuse sitzt.

In Fig. 4a ist schematisch gezeigt, dass der RFID-Transponder 3a einen Kontrolldatenspeicher 2a, z.B. einen elektronischen One-Time-Programmable-Baustein, aufweist, der lediglich ein einziges Mal beschrieben und nicht gelöscht oder wiederbeschrieben werden kann.

Der RFID-Transponder 3a weist ferner einen weiteren, lösch- und wiederbeschreibbaren Datenspeicher 40 auf. In einem Datenspeicherbereich 40a des Datenspeichers 40 ist eine Kennung des RFID-Transponders 3a hinterlegt, anhand derer mittels des RFID-Schreib-/Lesegeräts 7a durch den Rechner 14a der Vorrichtung 1a ausgelesen werden kann, ob der Flüssigkeitsbehälter 5a zur Verwendung in der Vorrichtung 1a autorisiert ist. Die Datenübertragung zwischen dem RFID-Schreib-/Lesegerät 7a und RFID-Transponder 3a erfolgt per Funk über eine Antenne 12a des RFID-Transponders 3a und einer Antenne 13a des RFID-Schreib-/Lesegeräts 7a.

Die Vorrichtung 1a weist einen Rechner 14a und einen Datenspeicher 16a auf und ist mittels eines lediglich in Fig. 4b gezeigten Pumpzähler 15a dazu eingerichtet, die Pumpvorgänge zu zählen. Anhand der Anzahl der Pumpvorgänge kann das Volumen an ausgegebener Flüssigkeit mittels des Rechners 14a bestimmt werden.

Bei dem nachfolgend erläuterten Beispiel sind in dem Flüssigkeitsbehälter 640 ml Seife 6a enthalten und als Kontrolldatenspeicher 2a ein 32 Bit-Speicher vorgesehen. Die Pumpe 17 ist derart eingerichtet, dass bei jedem Pumpvorgang 20 ml der Seife 6a ausgegeben werden.

Bei Ausgabe der Seife 6a aus der Vorrichtung 1a wird wie oben erläutert mittels der Vorrichtung 14a und des Pumpzählers das Volumen der ausgegebenen Seife 6a bestimmt. Immer wenn ein Pumvorgang durchgeführt worden ist und die 20 ml der Seife 6a ausgegeben worden sind, wird mittels des RFID-Schreib-/Lesegeräts 7 jeweilig ein Bit des Kontrolldatenspeichers 2 mit der Zahl "1" beschrieben. Aus dem Flüssigkeitsbehälter sind 640 ml der Seife 6a ausgegeben worden, wenn die 32 Bit des Kontrolldatenspeichers 2 vollständig beschrieben sind.

Der Rechner 14a der Vorrichtung 1a ist derart programmiert, dass eine Ausgabe der Seife 6a lediglich dann zugelassen wird, wenn der Kontrolldatenspeicher 2a nicht vollständig beschrieben ist.

Da die Vorrichtung 1a den Kontrolldatenspeicher 2a nach sukzessiver Ausgabe von je 20 ml der Seife 6a immer wieder beschreibt, können unter Verwendung des mit dem RFID-Transponders 3a versehenen Flüssigkeitsbehälters 5a lediglich die genannten 640 ml der Seife 6a ausgegeben werden. Selbst wenn der Flüssigkeitsbehälter 5a nach Abgabe der Seife 6a wieder aufgefüllt würde, könnten insgesamt nicht mehr als die 640 ml abgegeben werden. Dadurch wird verhindert, dass der Flüssigkeitsbehälter 5a unautorisiert wieder befüllt und die unautorisiert in den Flüssigkeitsbehälter 5a gegebene Seife 6a mittels der Vorrichtung 1a abgegeben werden kann.

In einem weiteren Ausführungsbeispiel wird zusätzlich zu der Speicherung im Kontrolldatenspeicher 2a in einem Vergleichsdatenspeicher 40b, der Datenspeicherbereich des Weiteren, lösch- und wiederbeschreibbaren Datenspeichers 40 ist, die Information über das Volumen der abgegebenen Seife 6a gespeichert. Um das Volumen genauer bestimmen zu können, wird in den Vergleichsdatenspeicher 40b jeweilig nach Ausgabe von 5 ml der Seife 6a eine entsprechend Information gespeichert.

Der Rechner 14a ist dazu vorgesehen, mittels eines Algorithmus die Information aus dem Kontrolldatenspeicher 2a mit derjenigen des Vergleichsdatenspeichers 40b zu vergleichen. Sollten die daraus bestimmbaren Längeninformationen einander entsprechen, d.h. die Längeninformation aus dem Vergleichsdatenspeicher 40b, die in 5 ml Schritten angelegt worden ist, liegt innerhalb der in 20 ml Schritten vorgesehenen Volumeninformation aus dem Kontrolldatenspeicher 2a, wird eine Seifenausgabe mittels der Vorrichtung 1 freigegeben. Andernfalls, d.h. wenn die Information im Vergleichsdatenspeicher 40b manipuliert worden ist, wird eine Ausgabe blockiert.

Alternativ kann vorgesehen sein, dass in dem Fall, dass die Information aus dem Kontrolldatenspeicher 2a derjenigen aus dem Vergleichsdatenspeicher 40b entsprich, eine der Information aus dem Kontrolldatenspepicher 2a entsprechende Volumeninformation in den Vergleichsdatenspeicher 40b geschrieben wird. Aus dem Flüssigkeitsbehälter 5a kann dann noch so viel Seife 6a abgegeben werden, wie anhand der Information aus dem Kontrolldatenspeicher 2a vorgesehen ist.

Es versteht sich, dass der Flüssigkeitsbehälter 5a anstatt mit der genannten Seife 6a auch mit Desinfektionsmittel gefüllt sein könnte.

## Patentansprüche

1. Vorrichtung zur Abgabe eines Tuchs, insbesondere eines Handtuchs, oder einer Reinigungsflüssigkeit, insbesondere von Seife oder Desinfektionsmittel, die dazu vorgesehen ist, eine Information zu einer in der Vorrichtung (1) angeordneten oder/und mittels der Vorrichtung (1) abgegebenen Menge des Tuchs oder der Reinigungsflüssigkeit zu speichern,
**dadurch gekennzeichnet,**
**dass** die Vorrichtung (1) dazu vorgesehen ist, die Information derart zu speichern, dass sie nach der Speicherung nicht veränderbar ist.

2. Vorrichtung nach Anspruch 1,
**dadurch gekennzeichnet,**
**dass** die Vorrichtung (1) zur Speicherung der Information in einem, vorzugsweise elektronischen, lediglich einmalig beschreibbaren Kontrolldatenspeicher (2) vorgesehen ist, wobei der Kontrolldatenspeicher (2) vorzugsweise Teil eines Funkchips (3), vorzugsweise eines RFID-Transponders, ist.

3. Vorrichtung nach Anspruch 1 oder 2,
**dadurch gekennzeichnet,**
**dass** der Kontrolldatenspeicher (2) ein Festwertspeicher, vorzugsweise ein One-Time-Programmable-Baustein, ist.

4. Vorrichtung nach einem der Ansprüche 1 bis 3,
**dadurch gekennzeichnet,**
**dass** die Vorrichtung (1) dazu vorgesehen ist, die Informationen zu der abgegebenen Menge des Tuchs oder der Reinigungsflüssigkeit in einem Vergleichsdatenspeicher (4) zu speichern, der vorzugsweise lösch- und wiederbeschreibbar ist.

5. Vorrichtung nach Anspruch 4,
**dadurch gekennzeichnet,**
**dass** die Vorrichtung (1) dazu vorgesehen ist, die in dem Kontrolldatenspeicher (2) und in dem Vergleichsdatenspeicher (4) gespeicherte Information zu der abgegebenen Menge automatisch miteinander zu vergleichen.

6. Vorrichtung nach einem der Ansprüche 1 bis 5,
**dadurch gekennzeichnet,**
**dass** die Vorrichtung (1) dazu vorgesehen ist, in dem Fall, dass sich die Mengenabgabeinformation des Kontrolldatenspeichers (2) und des Vergleichsdatenspeichers (4) nicht einander entsprechen, eine Abgabe des Tuchs oder der Reinigungsflüssigkeit mittels der Vorrichtung (1) zu verhindern oder die in dem Kontrolldatenspeicher (2) gespeicherte Information zu der Menge in dem Vergleichsdatenspeicher (4) zu speichern.

7. Vorrichtung nach einem der Ansprüche 1 bis 6,
**dadurch gekennzeichnet,**
**dass** die Vorrichtung (1) zur Aufnahme eines Tuch- oder Flüssigkeitsträgers (5), vorzugsweise einer das Tuch tragenden Rolle oder eines Flüssigkeitsbehälters, vorgesehen ist, wobei der Tuch- oder Flüssigkeitsträger (5) vorzugsweise mit dem Kontrolldatenspeicher (2) und ggf. dem Vergleichsdatenspeicher (4) versehen ist.

8. Vorrichtung nach einem der Ansprüche 1 bis 7,
**dadurch gekennzeichnet,**
**dass** die Vorrichtung (1) einen Dosierspender, vorzugsweise einen Seifen- und/oder Desinfektionsmittelspender, oder einen Handtuchspender, vorzugsweise einen Papierhandtuch- oder einen Stoffhandtuchspender, umfasst.

9. Tuch- oder Flüssigkeitsträger (5) zur Anordnung in einer Vorrichtung (1) zur Abgabe eines Tuchs oder einer Reinigungsflüssigkeit nach einem der Ansprüche 1 bis 8,
**dadurch gekennzeichnet,**
**dass** der Tuch- oder Flüssigkeitsträger (5) mit einem Kontrolldatenspeicher (2) zur Speicherung einer Information zu einer von dem Tuch- oder Flüssigkeitsträger (5) abgegebenen Menge des Tuchs oder der Reinigungsflüssigkeit versehen ist und der Kontrolldatenspeicher (2) vorgesehen ist derart, dass die gespeicherte Information nach ihrer Speicherung nicht veränderbar ist.

10. Tuch- oder Flüssigkeitsträger nach Anspruch 9,
**dadurch gekennzeichnet,**
**dass** der Tuch- oder Flüssigkeitsträger (5) einen Vergleichsdatenspeicher (4) umfasst, der vorzugsweise veränderbar ist.

11. Tuch- oder Flüssigkeitsträger nach Anspruch 9 oder 10,
**dadurch gekennzeichnet,**
**dass** der Tuch- oder Flüssigkeitsträger (5) vorgesehen ist derart, dass die Information mittels der Vorrichtung (1) in dem Kontrolldatenspeicher (2) und ggf. dem Vergleichsdatenspeicher (4) speicherbar ist.

12. Verfahren zur Abgabe eines Tuchs (6), insbesondere eines Handtuchs, oder einer Reinigungsflüssigkeit (6a), insbesondere von Seife oder Desinfektionsmittel, bei dem eine Information zu einer mittels der Vorrichtung (1) abgegebenen Menge des Tuchs oder der Flüssigkeit (6) gespeichert wird, **dadurch gekennzeichnet,**
**dass** die Information derart gespeichert wird, dass sie nach ihrer Speicherung nicht veränderbar ist.

13. Verfahren nach Anspruch 12,
**dadurch gekennzeichnet,**
**dass** die Information in einem, vorzugsweise elektronischen, lediglich einmalig beschreibbaren Kontrolldatenspeicher (2) und vorzugsweise einem, bevorzugt lösch- und wiederbeschreibbaren, Vergleichsdatenspeicher (4) gespeichert wird.

14. Verfahren nach Anspruch 12 oder 13,
**dadurch gekennzeichnet,**
**dass** die in dem Kontrolldatenspeicher (2) und in dem Vergleichsdatenspeicher (4) gespeicherte Information automatisch miteinander verglichen wird.

15. Verfahren nach Anspruch 14,
**dadurch gekennzeichnet,**
**dass** in dem Fall, dass sich die Mengenabgabeinformationen des Kontrolldatenspeicher (2) und des Vergleichsdatenspeichers (4) nicht entsprechen, eine Abgabe des Tuchs oder der Flüssigkeit (6) mittels der Vorrichtung (1) verhindert wird oder die in dem Kontrolldatenspeicher (2) gespeicherte Information zu der Menge in dem Vergleichsdatenspeicher (4) gespeichert wird.
